# EUROPEAN PATENT SPECIFICATION

(11) **EP 3 646 827 B1**
(45) Date of publication and mention of the grant of the patent: **28.06.2023**
(21) Application number: 17915783.9
(22) Date of filing: 13.10.2017
(51) Int. Cl.: A61F 5/00, A61M 25/01

(54) **GASTRIC DIVERTER AND ANTIBACTERIAL DIGESTIVE TRACT CATHETER THEREOF**
MAGENABLENKER UND ANTIBAKTERIELLER VERDAUUNGSTRAKTKATHETER DAFÜR
DÉRIVATION GASTRIQUE ET CATHÉTER DE TUBE DIGESTIF ANTIBACTÉRIEN ASSOCIÉ

(30) Priority: 27.06.2017 CN 201710499034
(43) Date of publication of application: 06.05.2020
(73) Proprietor: Hangzhou Tangji Medical Technology Co., Ltd., Hangzhou, Zhejiang 310052 (CN)
(72) Inventor: ZUO, Yuxing, Hangzhou, Zhejiang 310052 (CN); LU, Yan, Hangzhou, Zhejiang 310052 (CN)
(74) Representative: Viering, Jentschura & Partner mbB Patent- und Rechtsanwälte
(86) International application number: PCT/CN2017/105992
(87) International publication number: WO 2019/000704

(56) References cited:
- EP-A1- 2 561 840
- CN-A- 101 843 536
- CN-A- 103 479 454
- US-A1- 2004 107 004
- US-A1- 2010 256 775
- US-A1- 2011 004 146
- US-A1- 2013 030 351
- US-A1- 2016 135 976
- US-A1- 2017 119 567

## Description

### Field of the Invention

The present invention relates to the technical field of medical equipment. It is especially related to a gastric diverter and an antibacterial digestive tract catheter, which are used to treat endocrine diseases or reduce digestive tract diseases, such as inflammation, diabetes, pancreatic islet dysfunction and obesity.

### Background of the Invention

In recent years, people have paid more attention to diabetes. Diabetes is a common endocrine disease caused by fine dining and ingestion of more food, excessive nutrition intake and less exercising. Specifically, diabetes is a group of metabolic disorders characterized by high blood sugar, due to failure to produce enough insulin or respond to insulin properly or both. Serious long term complications of diabetes include cardiovascular disease, chronic kidney disease, and damage to the eyes and nerves. Other complications of diabetes such as obesity also pose a great threat to people's health. Among adults over 20 years old in China, the prevalence of diabetes is 9.7%, which means that 150 million people have diabetes in China. Treatment of diabetes involves maintaining a healthy diet, regular physical exercise, and medication treatment, which requires persistence. If patients stop their healthy diet, they will likely regain the weight, and even have rebound weight gain. Furthermore, constant medication treatment or insulin injection remain a financial burden and inconvenience in daily life.

Another therapy is gastric bypass surgery which has tremendous effects in curing type 2 diabetes and improving the symptoms of and obesity. In 2001, the International Diabetes Federation officially recommended that metabolic surgery (including gastric bypass surgery) should be considered as an appropriate treatment for obesity with type 2 diabetes. However, as a surgical procedure, gastric bypass surgery may leave surgical wound in human bodies, and involve the potential for complications which increase risk and mortality, such as bowel obstruction, anastomotic leakage, pulmonary embolism, deep vein thrombosis, portal vein thrombosis, and respiratory failure.

At present, treating diabetes by implanting a structure such as a gastric diverter in the gastrointestinal tract is also adopted in other countries. However, the gastric diverter is complicated in structure, and usually requires multiple wires to be pulled simultaneously to realize its function. Before operation, professional guidance is required. Lack of rich experience will likely lead to operational errors and increase the discomfort of patients. Due to its relatively difficult operation, high manufacturing cost and the technical barrier, wide-range promotion and application of the gastric diverter are difficult in the country. Therefore, it has become an urgent problem to be solved to provide a method for treating diabetes without much discomfort or a device with simple structure, operation convenience and low cost.

Although the existing gastric diverter products have achieved very good effects in treatment and reversal of diseases such as diabetes and weight loss, a small number of complications have been found. For example, hepatic abscess is a relatively serious complication. This limits the wide clinical application of this revolutionary product.

At present, there are some cases of treating diabetes by implanting a product similar to the gastric diverter structure in the gastrointestinal tract both at home and abroad. A flexible catheter made of a polymer material is implanted into the duodenum and generally fixed to the duodenal bulb through a small-sized metal stent. Currently, this product produced by GI Company in the U.S. has been used in clinical practice. There are also many similar designs. The stent, which is of a V-shaped or wavy structure, is fixed to the duodenal bulb by barbs. The risks of this design are that the barbs would penetrate into the duodenum and cause injury to the human digestive tract, and that the barbs would easily injure the esophagus and cause esophageal laceration when the stent is taken out. If the catheter accidentally falls into the distal end of the duodenum, the jejunum, or even the colon, it may easily cause intestinal obstruction. Therefore, a better design is needed to prevent these risks.

A further digestive tract catheter of a gastric diverter is known from US 2010/256 775 A1

### Technical Problem

In order to solve the above technical problems, the objective of the present invention is to provide a gastric diverter and an antibacterial digestive tract catheter thereof. A digestive tract isolation catheter made of an antibacterial material may reduce the inflammation of an intestinal tract as much as possible, thereby improving human metabolic diseases and avoiding incidence of complications such as hepatic abscess and pancreatitis. Therefore, a safer and more effective medical device for treating the metabolic diseases such as diabetes is provided.

### Solution to Problems

### Technical Solution

In view of the above problems in the prior art and after careful study, inventors of the present invention add antibacterial materials such as nano silver ions, silver particles, nano silver, nano copper ions, copper particles, vanillin, ethyl vanillin compounds or chitosan into a membrane tube polymer material. These materials are easily added into polymer materials, may not affect the physical properties of the materials, and would not cause harm to the human body.

The present invention relates to an antibacterial digestive tract catheter as set out in claims 1-8.

Preferably, the membrane tube contains a polymer material, an antibacterial material and a developing material. The raw materials are uniformly mixed and then subjected to blow molding, extrusion, mold pressing or hot press forming to form a thin film or a tubular film.

Preferably, the membrane tube is a thin film, a tubular film or a thin-wall tube made of the polymer material with the outer surface brushed with the antibacterial material. Preferably, the polymer material should have a good biocompatibility, and can be retained in the human body for a long time without any adverse effect on the human body. The long-time retention refers to retention in the human digestive tract for three months or longer time.

Preferably, the polymer material should be flexible enough to avoid adverse stimulations to a gastrointestinal mucous membrane, and a patient would not feel uncomfortable after the implantation. Even if slight discomfort may be felt by someone, it will disappear by itself in a short time.

Preferably, the polymer material should have good chemical and biological stability, and chemical changes shall not occur and soluble substances unfavorable for the human body shall not be produced under the influence of various strong acids and strong alkalis as well as biological enzymes in the digestive tract.

Preferably, the polymer material should have good processibility, and may be processed into a thin film or a tubular film with a thickness of 0.001-0.3 mm.

Preferably, the polymer material may be any one of or a compound of several of polyethylene, fluorine polymer, polyurethane, silica gel, TPU and the like that meet the above requirements.

Preferably, the antibacterial material should have good biocompatibility and be non-toxic and harmless to the human body and environmentally friendly.

Preferably, the antibacterial material should have an obvious antibacterial and inhibitory effect on pathogenic microorganisms and maintain the antibacterial property for a relatively long time.

Preferably, the antimicrobial material should have excellent physical properties and certain strength and flexibility in human tissues.

Preferably, the antibacterial material should be self-cleaning and environmental friendly, and convenient to use, and a self-degrading capacity is preferred.

Preferably, a developing material may be added into the catheter implanted in the digestive tract to determine a retention position of the catheter implanted in the digestive tract in the human body, and to determine whether twisting and knotting exist and the like.

Preferably, the developing material should have good biocompatibility, be nontoxic and harmless to the human body, and does not affect the physical shape of the catheter implanted in the digestive tract after being added in a proper amount, and the catheter may still remain flexible.

Preferably, the developing material may be one or more of barium sulfate, bismuth carbonate and a tungsten compound.

Preferably, the polymer material, the antibacterial material and the developing material are uniformly mixed with each other, and may be processed into a film-like tubular material by a well-known polymer processing technology such as blow molding, extrusion and mold pressing. The film is 0.001-0.3 mm in thickness. The diameter of the tubular material is 10-35 mm.

The antibacterial digestive tract catheter may be implanted into the digestive tract. The digestive tract includes an esophagus, a stomach, a duodenum, a jejunum, an ileum and the like, so that different positions of the implantable catheter may correspond to the esophagus, the stomach, duodenum, the jejunum, the ileum and the like, respectively. Since different positions of the catheter have different functions, different effects may be exerted on different parts of the digestive tract.

Preferably, the stent (32) is a mesh tube woven from a weaving wire (103), and the weaving wire is a biocompatible elastic wire, which may be a metal wire, a polymer material wire or a degradable material wire.

Preferably, the stent (32) includes an upper stent section (101) and a lower stent section (102), and the outer diameter of the upper stent section (101) is 5-15 mm greater than the lower stent section (102) by 5-15 mm.

Preferably, the stent (32) is provided with a developing ring (106).

Preferably, the stent (32) is provided with a withdrawal wire (105).

A gastric diverter includes a shell, a release body, a pushing component and the above-mentioned antibacterial digestive tract catheter.

The shell (1) is tubular. One end of the shell (1) has a first opening (11), and the other end of the shell (1) has a second opening (13). A to-be-released folded catheter is arranged in the shell (1). The release body (2) is arranged at the first opening (11) and connected to one end of the membrane tube (31), and is made of a material that may be digested and absorbed or dissolved by the human intestinal tract. The pushing component includes an inner tube (41), a middle tube (42) and an outer tube (43) which are arranged in a sleeved manner in sequence and may move relative to each other. One portion of the inner tube (41) is located in the shell (1) and connected with the release body (2). One end of the middle tube (42) extends into the shell (1) through the second opening (13), and is fixedly provided with a stopper piston (12) which is located in the shell (1) and configured for abutting against the membrane tube (31). The outer tube (43) is located outside the shell (1), with one end fixedly connected to the second opening (13). The inner tube (41) moves towards an operator along its axial direction, so as to enable the release body (2) to be separated from the shell (1). The inner tube (41) and the middle tube (42) drive the membrane tube (31) to be separated from the shell and spread and then released at designated positions of the human intestinal tract.

### Beneficial Effects of the Present Invention

### Beneficial Effects

Because of the above technical solutions of the present invention, the antibacterial digestive tract catheter may be implanted into the digestive tract to treat endocrine diseases such as diabetes, pancreatic islet dysfunction and obesity, or lower digestive tract diseases such as inflammation. Compared with the prior art, the catheter of the present invention has a certain antibacterial effect and can effectively avoid or reduce the incidence of inflammation in the digestive tract, such as hepatic abscess and pancreatitis. Furthermore, the present invention has the advantages of simple material structure, simple manufacturing process, low cost, high production speed, quality assurance, manufacturing convenience and the like. Meanwhile, after implantation, it is comfortable and compliant, and will not cause harm to human tissues.

### Brief Description of the Drawings

Fig. 1 is a structural sectional diagram of an embodiment of the present invention;
Fig. 2 is an overall structural schematic diagram of an embodiment of the present invention;
Fig. 3 is a sectional view of a release body of one structure of an embodiment of the present invention;
Fig. 4 is a sectional view of a release body of another structure of an embodiment of the present invention;
Fig. 5 is a structural schematic diagram of an implantable catheter of the present invention;
Fig. 6 is a structural schematic diagram of an implementation of a stent of the present invention;
Fig. 7 is a structural schematic diagram of another implementation of a stent of the present invention; and
Fig. 8 is a structural schematic diagram of a further implementation of a stent of the present invention.

### Reference numerals in the drawings:

(1): shell; (11): first opening; (12): stopper piston; (13): second opening; (14): screw cap; (2): release body; (22): inner core; (221): membrane tube connection portion; (222): main body portion; (23): housing; (231): ring step; (31): membrane tube; (32): stent; (41): inner tube; (411): damping tube; (412): second extending end; (42): middle tube; (421): first extending end; (43): outer tube; (44): first handle; (45): Luer taper; (46): second handle; (101): upper stent section; (102): lower stent section; (103): weaving wire; (104): number of heads; (105): withdrawal wire; and (106): developing ring.

### Detailed Description of the Invention

### Best Mode for Carrying out the Invention

The embodiments of the present invention are described in detail below, and the examples of the embodiments are illustrated in the drawings, wherein the same or similar reference numerals refer to the same or similar elements or elements having the same or similar functions throughout. The embodiments described below with reference to the drawings are exemplary and intended to be explanatory of the present invention and are not to be construed as limiting the present invention.

In the description of the present invention, it should be understood that directions or position relationships indicated by the terms, such as "center", "longitudinal", "transverse", "length", "width", "thickness", "up", "down", "front", "rear", "left", "right", "vertical", "horizontal", "top", "bottom", "inside", "clockwise" and "anticlockwise", are directions or position relationships as shown in the drawings, and are only to facilitate and simplify the description of the present invention instead of indicating or implying that devices or elements indicated should have specific directions and be constructed and operated at the specific directions, so that these terms may not be construed as limiting the present invention.

In addition, the terms "first" and "second" are only for description, but not construed as indicating or implying relative importance or impliedly indicating the number of technical features indicated. Therefore, features defined by "first" and "second" may explicitly indicate or impliedly include one or more features. In the description of the present invention, unless otherwise specifically specified, the term "multiple" means that the number is equal to or more than 2.

In present invention, unless otherwise clearly specified and defined, the terms "mounted", "coupled", "connected" and "fixed" shall be general understandings. For example, it may be fixed connection, detachable connection, or integrated connection. It may be mechanical connection, electrical connection, direct connection, or indirect connection through an intermediate, or communication of insides of two elements. Those of ordinary skill in the art can understand specific meanings of the above-mentioned terms in the present invention according to specific situations.

In the present invention, unless otherwise clearly specified and defined, a situation that a first feature is "above" or "below" a second feature may include direct contact of the first and second features, and may also include a situation that the first and second features are in contact through other features therebetween instead of direct contact. In addition, a situation that the first feature is "on", "above" and "on the upside of" the second feature includes situations that the first feature is right above and in the oblique above the second feature, or only represents that the horizontal height of the first feature is greater than that of the second feature. A situation that the first feature is "under", "below" and "underneath" the second feature includes situations that the first feature is right below and in the oblique below the second feature, or only represents that the horizontal height of the first feature is less than that of the second feature.

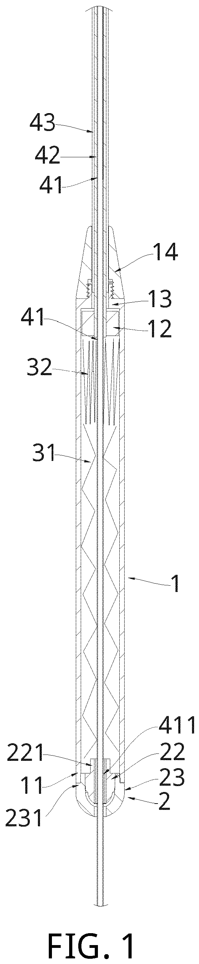

A gastric diverter as shown in Fig. 1 includes a shell (1), a release body (2), a pushing component and an implantable catheter. The shell (1) is tubular. The shell (1) is tubular and has a first opening (11) at one end and a second opening (13) at the other end. The folded implantable catheter to be released is disposed in the shell (1). The release body (2) is disposed at the first opening (11), connected to one end of the membrane tube (31), and made of a material that is able to be absorbed or dissolved in human intestines. The pushing component includes an inner tube (41), a middle tube (42) and an outer tube (43) sequentially set and able to move relative to each other. The inner tube (41) is partially located in the shell (1) and connected to the release body (2). An end of the middle tube (42) extends into the shell (1) through the second opening (13) and is fixedly provided with a piston stopper (12) located in the shell (1) and configured for pushing the membrane tube (31). The outer tube (43) is located outside the shell (1) and has an end fixedly connected to the second opening (13). The inner tube (41) moves axially toward an operator to disengage the release body (2) from the shell (1). The inner tube (41) and the middle tube (42) drive the membrane tube (31) to move out of the shell and spread and to release at designated positions of the human intestines.

The inner tube (41) moves and drives the release body (2) to move, thereby pulling the membrane tube (31) to allow it to spread gradually. The middle tube (42) moves along its axial direction and drives the stopper piston (12) to press against the membrane tube (31) (namely press against the end, which is not connected to the release body (2), of the membrane tube (31)) to push the membrane tube (31) out of the shell (1).

A protrusion is provided outside the second opening (13) of the shell (1) and has a screw cap (14) screwed thereon. The outer tube (43) passes through the screw cap (14) and is fixedly connected to the second opening (13) of the shell (1). Specifically, the outer tube (43) can be fixedly connected to the second opening (13) by sticking, thermal shrinkage, or other manners.

In this embodiment, the shell (1) and each part of the pushing component are made of one or more composite materials of polyurethane, polyethylene, and fluoropolymer. Aside from good support performance, pushability, toughness and a smooth surface to be smoothly pushed via an endoscope or in the tracts of the human body, also these materials are not easy to be bent and can be well controlled.

Specifically, in the use of the gastric diverter of the present embodiment, a guide wire is passed through the inner tube (41), the operator passes the entire device through the human mouth. The shell (1) is guided by the guide wire in cooperation with the gastroscope to the duodenum close to the pylorus and/or an upper part of the pylorus. The guide wire and the pushing component guide and support the movement of the inner tube (41) in the esophagus and gastrointestinal tract, so that the shell (1) and the membrane tube (31) can successfully reach designated positions. Then, the middle tube (42) and the inner tube (41) in the pushing component are operated to release the membrane tube (31) in the shell (1) into the gastrointestinal tract, and the inner tube (41) is operated to move the release body (2) to unfold the folded membrane tube (31). After the release body (2) made of a material that can be digested and absorbed in the human intestinal tract enters the intestines, it can be dissolved or decomposed in a short period of time by digestive juices or water, and then be digested and absorbed in the intestines, thereby completing the release of the membrane tube (31). In this way, a thin-film guide tube forms on the intestinal tissues to prevent and slow down nutrient absorption in the intestinal tract, and regulate the secretion of digestive enzymes, thereby regulating blood sugar and lipid levels and body weight, and avoiding the occurrence of diabetes. The release body (2) is made of a material which meets a biological safety requirement, can be dissolved or decomposed in the human body, and has no toxicity or side effects for the human body. The material may be, for example, one or more compounds of an edible gelling agent, a soy protein powder, starch, a polysaccharide compound, glycerin, branched or amylopectin, and the like. During formulation, the one or more compounds are added, mixed with water and a fat-soluble substance, and then solidified in a corresponding mold, to form a hard solid structure. Moreover, the release body (2) can also be made of a material which can be dissolved but not absorbed in the human body, and completely eliminated from the human body, without toxicity or side effect.

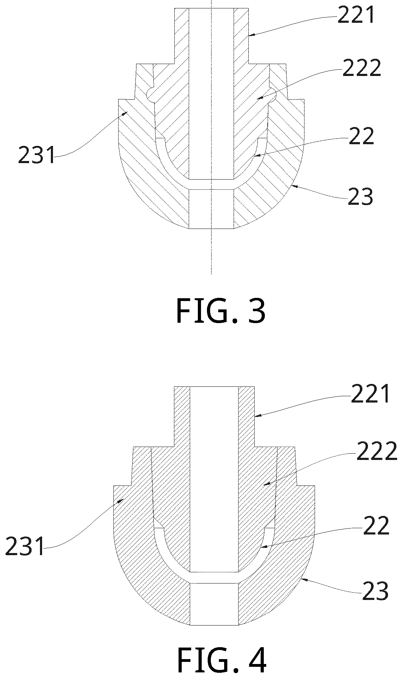

As shown in Figs. 3 and 4, the release body (2) includes an inner core (22) and a housing (23). The inner core (22) has a main body portion (222) and a membrane tube connection portion (221) connected to the one end of the membrane tube (31). The housing (23) covers the main body portion (222) outside, is connected to the first opening (11) and is easily broken under force.

Preferably, referring to Figs. 3 and 4, a surface of the main body portion (222) of the inner core (22) and a surface of the housing (23) facing a release direction of the membrane tube (31) are round smooth surfaces, which facilitates the movement of the release body (2) in the human intestinal tract.

Specifically, referring to Figs. 3 and 4, the main body portion (222) has an outer contour in a spherical or hemispherical shape. The housing (23) has an outer contour matches the outer contour of the main body portion (222) in shape, and covers the main body portion (222) outside. In this embodiment, the main body portion (222) has the outer contour in a hemispherical shape, and the housing (23) has the outer contour in a hemispherical shape. In addition, the main body portion (222) and the housing (23) can be approximately shaped like a sphere or hemisphere.

Preferably, referring to Figs. 3 and 4, the main body portion (222) has the outer contour in a hemispherical shape, the membrane tube connection portion (221) is connected to a planar portion of the main body portion (222), and the housing (23) is in a hemisphere shape and covers the main body portion (222) outside a hemispherical surface thereof. A ring step (231) is formed on the periphery of an end surface of the hemispherical housing (23), and is abutted against the second opening (13) of the shell (1).

Specifically, referring to Figs. 3 and 4, in the above embodiment, the inner core (22) is physically connected to the housing (23) by, for example, screwing or engaging as shown. Specifically, Fig. 3 shows the screwed inner core (22) and the housing (23). The inner core (22) and the housing (23) both include a portion in an approximately spherical shape and a portion in a cylindrical shape, and have threads engaged with each other on the portions in a cylindrical shape of the inner core (22) and the housing (23). Fig. 4 shows the engaged inner core (22) and housing (23). An outer surface of the inner core (22) and an inner wall of the housing (23) are respectively provided with fasteners which can be engaged to each other. It should be noted that the connection manner between the inner core (22) and the housing (23) is not limited to screwing or engaging, and can be a taper fit or other connection manners. For example, the housing (23) and the inner core (22) are taper-fitted to each other. Other similar structures are not described in detail herein.

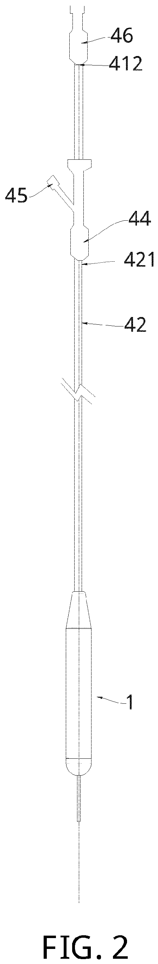

Preferably, referring to Fig. 2, an end of the inner tube (41) sequentially passes through the membrane tube connection portion (221) and the main body portion (222) of the inner core (22), and the housing (23), and extends out of the first opening (11) to allow an endoscope to inspect the intestinal tract. In this embodiment, the inner tube (41) forms a tight fit or an interference fit with the membrane tube connection portion (221) and the main body portion (222) of the inner core (22), and the housing (23), such that the inner tube (41) can drive the release body (2) to move along the release direction of the membrane tube (31) and the membrane tube (31) to be unfolded and to extend out of the shell (1). The membrane tube connection portion (221), the main body portion (222) and the housing (23) can be connected to the inner tube (41) in different manners.

Preferably, referring to Fig. 2, a damping tube (411) is sleeved on a portion of the inner tube (41) passing out of the inner core (22). In this embodiment, a tight fit is formed between the inner tube (41) and the inner core (22) via the damping tube (411). The damping tube (411) is configured for increasing a sliding friction between the release body (2) and the inner tube (41), thereby enhancing the capability of the inner tube (41) to drive the release body (2) to move.

In the present embodiment, the damping tube (411) can be made from one or more elastic materials of polyurethane, silicone, and TPE, with a surface subjected to physical or chemical processing to increase friction. Accordingly, before the membrane tube (31) is released, the damping tube (411) maintains a relatively static state between the release body (2) and the inner tube (41).

Preferably, as shown in Fig. 2, the other end of the membrane tube (31) is fixedly connected to the stent (32), and the stent (32) is an elastic memory alloy braided stent in a mesh tube form. Specifically, the stent (32) can be made of a nickel titanium alloy, stainless steel, other elastic metal or other qualified memory alloys, meeting a biocompatibility requirement and suitable for long-term implantation in the human body. The stent (32) is regularly arranged in the shape of a rhombus, hexagon, or honeycomb, and placed in the duodenal bulb. The stent (32) is elastic and can vary in shape with the peristalsis of the intestinal tract. Also it has a certain supporting force and can be fixed in the duodenal bulb.

Specifically, during the operation of the middle tube (42), while the middle tube (42) drives the piston stopper (12) to push the stent (32) and the membrane tube (31) outward from the shell (1), the operator has to observe the stent (32) in the shell (1) via the endoscope. When the front end of the stent (32) is about to be out of the shell (1), the operation of the middle tube (42) should be stopped immediately. At the same time, the operator has to adjust the position of the shell (1) so that the stent (32) can be fixed in the duodenal bulb when leaving the shell (1). The stent (32) is elastic and can vary in shape with the peristalsis of the intestinal tract. Also it has a certain supporting force and can be fixed in the duodenal bulb.

Specifically, in the above embodiment, the structure used to operate the movement of the middle tube (42) and the inner tube (41) includes a first extending end (421) and a first handle (44), a Luer taper (45), a second extending end (412) and a second handle (46). The middle tube (42) has the first extending end (421) extending out of the outer tube (43), the first extending end (421) is connected to the first handle (44), the first handle (44) is provided with the Luer taper (45) communicating the outside with the inside of the middle tube (42), the inner tube (41) has the second extending end (412) extending from the first extending end (421), and the second extending end (412) is connected to the second handle (46).

Specifically, in the process of operating the middle tube (42) and the inner tube (41) to release the membrane tube (31), the operator can add a certain amount of water or saline through the Luer taper (45). As the water or saline enters the membrane tube (31) through a gap between the middle tube (42) and the inner tube (41), the release of the membrane tube (31) in the duodenum and the dissolution or decomposition of the release body (2) can be accelerated. When the membrane tube (31) is released, the membrane tube (31) and the release body (2) move from the duodenum towards the jejunum with the peristalsis of the intestinal tract for about 1 to 10 minutes. At the same time, as the saline flows towards the membrane tube (31) along the gap between the inner tube (41) and the middle tube (42), the movement of the membrane tube (31) and the release body (2) towards the jejunum and the release of the membrane tube (31) are also accelerated. The membrane tube (31) may contain a developing material, such as barium sulfate, bismuth carbonate, and tungsten, which can be developed under X rays. The release body (2) may also contain a developing material so as to provide its position under X rays, thereby facilitating the operation.

The present invention can be operated as follows. Firstly, the inner tube (41) is pulled by and towards the operator, such that when the second opening (13) of the shell (1) is pressed against the housing (23), the ring step (231) of the housing (23) is disconnected to separate the release body (2) from the shell (1). Then, the inner tube (41) is operated to move the release body (2) away from the operator (i.e. into the body of a patient), such that one end of the membrane tube (31) extends out of the shell (1) and drives the membrane tube (31) to be unfolded. In this case, the middle tube (42) is then operated to drive the piston stopper (12) to move. The piston stopper (12) pushes the stent (32) on the other end of the membrane tube (31) in the shell (1) to push the whole membrane tube (31) out of the shell (1). It should be noted that when the membrane tube (31) is completely unfolded and the piston stopper (12) pushes the stent (32) to move to the first opening (11) of the shell (1) (when the membrane tube (31) is about to be completely released), the stent (32) has to arrive at a specified release position in the intestinal tract (an appropriate position near the duodenum of the stomach pylorus and/or the upper portion of the stomach pylorus in the stomach of the body). Then, the second handle (46) is pushed in a direction away from the operator so that the stent (32) is completely detached from the shell (1) and fixed in the duodenal bulb. The stent (32) is elastic and can vary in shape with the peristalsis of the intestinal tract. Also, it has a certain supporting force and can be fixed in the duodenal bulb. The unfolded membrane tube (31) may be placed in the digestive tract for 1 to 12 months, and the duration can be adjusted according to disease conditions or actual conditions.

In this embodiment, there is still another way of operation described as follows. When the middle tube (42) and the inner tube (41) are simultaneously operated to move the piston stopper (12) to push one end of the membrane tube (31) in the shell (1), the inner tube (41) moves to drive the release body (2) to move, and the release body (2) drives the stent (32) on the other end of the membrane tube (31). In this way, the membrane tube (31) is unfolded and extends out of the shell (1). Finally, the whole membrane tube (31) is pushed out of the shell (1) at a specified release position in the intestinal tract.

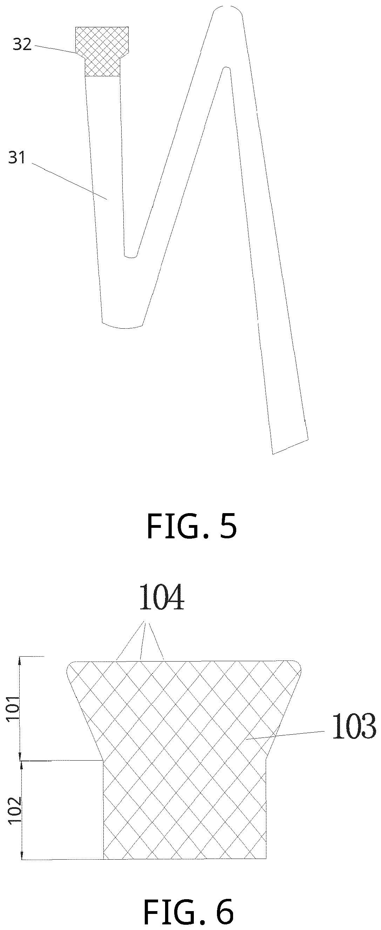

As shown in Fig. 5, the implantable catheter includes the membrane tube (31). One end of the membrane tube (31) is connected to the stent (32). The membrane tube (31) is made of an antibacterial material, with an outer diameter of 10-35 mm and a wall thickness of 0.01-0.1 mm. The membrane tube (31) and the stent (32) are fixedly connected in a suturing, hot pressing, ultrasonic welding or laser welding manner.

Preferably, the catheter contains a polymer material, an antibacterial material and a developing material. The raw materials are uniformly mixed and then subjected to blow molding, extrusion, or mold pressing to form a thin film or a tubular film. Preferably, the polymer material is any one or several of polyethylene, fluorine polymer, polyurethane and silica gel. Preferably, the antibacterial material is one or more of nano silver, nano copper, vanillin, an ethyl vanillin compound or chitosan. Preferably, the developing material is one or more of barium sulfate, bismuth carbonate and a tungsten compound.

Preferably, the outer diameter of the membrane tube according to the present invention may be 10-40 mm, preferably 15-35 mm, more preferably 15-30 mm. Within this range, the membrane tube (31) may be fully applicable to the human digestive tract and not injure the human tissues.

Preferably, the length of the membrane tube (31) according to the present invention may be 200-1,500 mm, preferably 200-1,300 mm, more preferably 300-1,200 mm. Within this range, the catheter according to the present invention may be implanted into any position of the digestive tract and define an acting position.

Preferably, the wall thickness of the membrane tube (31) according to the present invention may be 0.001-0.3 mm, preferably 0.005-0.05 mm, more preferably 0.01-0.03 mm. Within this range, the flexibility of the catheter according to the present invention may be fully guaranteed, and enough strength is maintained.

The implantable catheter may be implanted into the digestive tract. The digestive tract includes an esophagus, a stomach, a duodenum, a jejunum, an ileum and the like, so that different positions of the implantable catheter may correspond to the esophagus, the stomach, the duodenum, the jejunum, the ileum and the like, respectively. Since the catheter has different functions at different positions, different effects may be exerted on different parts of the digestive tract. The implantable catheter may have the function of isolating food in the intestinal tract and changing the physiological flowing direction of the food. After the catheter is implanted for a period of time, the insulin resistance of the body of the patient disappears. Furthermore, the flowing mode of the food may also promote the insulin secretion in the body of the patient, reduce apoptosis of pancreatic islet cells to multiply the pancreatic islet cells, recover pancreas pancreatic islet functions and cure the metabolic diseases such as diabetes and obesity. The implantable catheter has a tensile modulus of more than 250 MP and an elongation of more than 230 percent. The material is flexible. Nausea, vomiting, abdominal pain and other phenomena caused by the implantation in the body can be significantly reduced.

The implantable catheter according to the present invention may be implanted into the digestive tract to treat endocrine diseases, such as diabetes, pancreatic islet dysfunction and obesity, or lower digestive tract diseases, such as inflammation. In comparison with conventional technology, the implantable catheter of the present invention has the advantages of simple structure, good structural applicability, simple manufacturing process, low cost, high production speed, quality assurance, convenience in manufacturing and the like. Furthermore, after implantation, the implantable catheter is comfortable and compliant, and will not cause harm to human tissues.

In addition, the barium sulfate contained in the implantable catheter of the present invention is visible under X-ray and the like. In addition, the implantable catheter of the present invention may be connected to the stent. After the catheter is connected to the stent, stimulation to the digestive tract can be reduced, and it is safer and more convenient to lift, pull, adjust or withdraw the stent.

In one implementation of the catheter of the present invention, nano silver is selected as the antibacterial material. Nano silver powder is added into polytetrafluoroethylene material powder according to an adding ratio of 1/10000 to 1/1000 percent by weight, and then an auxiliary such as alcohol is added. The two materials are fully and uniformly mixed. The mixture is put into a special preparation device to manufacture a tube blank through heating, pressurization and other processes, and a heating temperature is about 300 DEG C. Then, the tube blank is put into a designed extrusion mold for secondary forming of a catheter. The tube blank is put into a designed iron rod, and the iron rod is placed into a heating furnace with ventilation to volatilize the solvent such as the alcohol and solidify the tube blank. The processed catheter is put into a thermal treatment furnace again for sizing. The obtained catheter is a highly-biocompatible flexible thin-wall tube with a diameter of 10-40 mm and a wall thickness of 0.01-0.1 mm, and can be retained for a long time in the human body without adverse effects on the human body. The long-time retention refers to retention in the human digestive tract for three months or longer time.

Preferably, a developing material may also be added into the material to determine a position for catheter implantation in clinical use, determine whether twisting and knotting exist and the like.

Preferably, the implantable catheter should have good chemical and biological stability, and chemical changes shall not occur and soluble substances unfavorable for the human body shall not be produced under the influence of various strong acids and strong alkalis as well as biological enzymes in the digestive tract.

Preferably, the catheter has an obvious antibacterial and inhibitory effect on pathogenic microorganisms, maintains the antibacterial property within relatively long time, is non-toxic and harmless to the human body, and is environmentally friendly.

Preferably, the catheter has good physical properties, and has certain strength and flexibility in the human tissues.

In another implementation of the present invention, chitosan is selected as an antibacterial material. The chitosan is added into a PE master batch according to an adding ratio of 5-25 percent by weight to prepare a chitosan-containing PE master batch. Linear low-density polyethylene (LLDPE) is selected as a base material. The chitosan-containing PE master batch is added into LLDPE plastic particles for uniform mixing. A thin-wall tube is processed by an extrusion process at a preferable processing temperature of 100 to 130 DEG C. A gas pressure needs to be controlled in the extrusion process to keep a stable inner diameter. A catheter prepared from the obtained antibacterial material is a flexible thin-wall tube that has a wall thickness of 0.01-0.1 mm and a diameter of 10-40 mm, may not generate adverse effects to the digestive tract and has a relatively good bacteriostatic effect.

Preferably, a developing material may also be added into the material to determine the position of the catheter in the human digestive tract. Preferably, the developing material uses barium sulfate. The specific adding amount is 10 to 35 percent by weight.

The antibacterial digestive tract catheter prepared by the above implementation of the present invention is subjected to a bacteriostatic test. The same material (but with no bacteriostatic material) is used to prepare a film-like catheter of the same size. A strip material of 15 mm*30 mm is selected and placed into an agar culture medium. They are placed in two culture dishes respectively and cultured for 48 hours under the same culture conditions. It can be found that the total number of colonies cultured by using the digestive tract catheter used in the present invention is less than that of colonies cultured by using the sample without the bacteriostatic material by 50 percent or above.

As shown in Fig. 5, the membrane tube (31) is connected to one end of the stent (32) in one or a combination way of several of suturing, hot pressing, ultrasonic welding and laser welding.

As shown in Figs. 6 to 8, the present embodiment provides a digestive tract stent (32) divided into an upper stent section (101) and a lower stent section (102). The diameter of the upper stent section (101) is preferably 25-40 mm, which is greater than that of the duodenal bulb. The diameter of the lower stent section (102) is preferably 15-25 cm which is close to or slightly greater than that of the duodenum. The diameter of the upper stent section (101) shall be greater than that of the lower stent section (102). Preferably, the diameter of the upper stent section (101) is 5-15 mm greater than that of the lower stent section (102).

Preferably, the stent may be of a funnel shape as shown in Fig. 6, namely the upper stent section (101) is of an inverted trapezoidal shape, and the diameter of the upper stent section (101) is greater than that of the lower stent section (102). Preferably, the diameter of the upper stent section (101) is 25-40 mm, which is greater than that of the upper section of the duodenum. The diameter of the lower stent section (102) is 15-25 mm, which is equivalent to that of the upper section of the duodenum. The diameter of the upper stent section (101) is 5-15 mm greater than that of the lower stent section (102). A rounded rectangle of the upper stent section (101) is fixed at the duodenal bulb. The inverted trapezoidal design can realize smooth transition to maximally avoid an injury caused by the long-term implantation to a digestive tract wall, and provide the highest supporting force. The lower section is connected to the membrane tube. The membrane tube (31) extends to the lower section of the duodenum or the upper section of the jejunum.

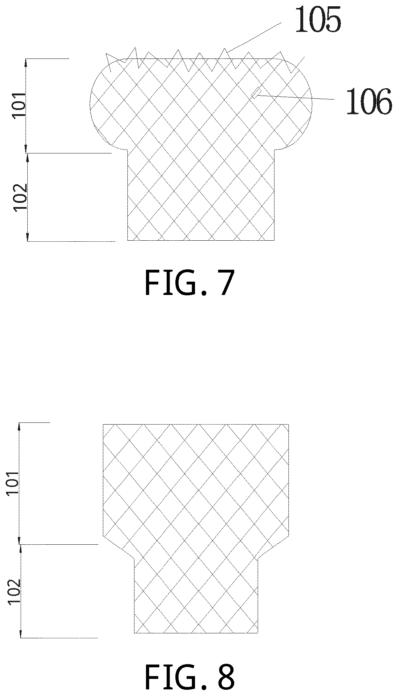

Preferably, the stent (32) may also be of a round ball shape as shown in Fig. 7, namely the upper stent section (101) is elliptical, and the diameter of the upper stent section 101 is greater than that of the lower stent section (102). Preferably, the diameter of the upper stent section (101) is 25-40 mm, which is greater than that of the upper section of the duodenum. The diameter of the lower stent section (102) is 15-25 mm, which is equivalent to that of the upper section of the duodenum. The diameter of the upper stent section (101) is 5-15 mm greater than that of the lower stent section (102). A round ball of the upper stent section (101) is fixed at the duodenal bulb. The arc-shaped design can maximally avoid an injury caused by the long-term implantation to a digestive tract wall, and arc transition can provide a high supporting force. The lower stent section (102) is connected to the membrane tube (31). The membrane tube (31) extends to the lower section of the duodenum or the upper section of the jejunum.

Preferably, the stent (32) may also be of a wine glass shape as shown in Fig. 8, namely the upper stent section (101) is a rounded rectangle, and the diameter of the upper stent section (101) is greater than that of the lower stent section (102). Preferably, the diameter of the upper stent section (101) is 25-40 mm, which is greater than that of the upper section of the duodenum. The diameter of the lower stent section (102) is 15-25 mm, which is equivalent to that of the upper section of the duodenum. The diameter of the upper stent section (101) is 5-15 mm greater than that of the lower stent section (102). A rounded rectangle of the upper stent section (101) is fixed at the duodenal bulb. The rounded rectangle design maximizes the contact area between the stent and the duodenal bulb to maximally avoid an injury caused by the long-term implantation to a digestive tract wall. The lower stent section (102) is connected to the membrane tube. The membrane tube extends to the lower section of the duodenum or the upper section of the jejunum.

Preferably, the upper stent section (101) of the stent (32) is 5-15 mm high, and the lower stent section (102) is also 5-15 mm high. The specific heights may be slightly adjusted according to a specific physiologically anatomical structure of the human body.

The stent (32) is regularly arranged in the shape of a rhombus, a hexagon and a honeycomb and placed in the duodenal bulb. The stent (32) is elastic and can vary in shape with the peristalsis of the intestinal tract. Also it has a certain supporting force and can be fixed in the duodenal bulb.

A weaving wire (103) of the stent (32) may be a wire of a metal material, a polymer material or a degradable material. The metal wire material may be a stainless steel wire and a nickel-titanium alloy wire. The polymer material may be PE (polyethylene), PU (polyurethane) and PTFE (polytetrafluoroethylene). The degradable material may be PPDO (poly(p-dioxanone)), PLLA (polylactic acid), PGA (polyglycolic acid) and the like. The material meets a biocompatibility requirement, suitable for long-term implantation in the human body. It is tough and elastic, has a certain supporting force in the digestive tract, and is connected to the membrane tube (31) to prevent slippage.

Preferably, the weaving wire (103) uses a nickel-titanium alloy. The nickel-titanium alloy wire preferably meets the long-term implantation requirement and has a preferable austenitic phase change point of 0 to 10 degrees.

The stent (32) is woven from a wire on a mold and then subjected to heat setting. A set product may be of a structure as shown in Figs. 6 to 8 or a similar structure meeting the human anatomy and physiology. The stent (32) has a certain supporting force and compression force in both a transverse direction and a longitudinal direction and can vary in shape with the peristalsis of the human intestinal tract. Also it has a uniform supporting force and can be fixed at the duodenal bulb, and may recover to a heat-set shape and be fixed at the duodenal bulb 113.

Specifically, a weaving route of the stent (32) is of a convolution body shape rotating around the middle axis. The rotating direction of the convolution body may be a clockwise rotating direction or an anticlockwise rotating direction when overlooked from the head end portion.

As shown in Fig. 7, the stent (32) has a withdrawal wire (105). The withdrawal wire (105) is sutured to the outermost circle of the upper stent section (101) of the stent (32). The stent (32) may be taken out and withdrawn from the body through a special withdrawer or a nipper. When the withdrawal wire (105) is pulled in, the stent (32) overall retracts and enters a transparent cap of the endoscope or a specific withdrawal device, and is taken out from the human digestive tract with the endoscope. The withdrawal wire (105) may be a biocompatible material such as PE, nylon and terylene.

Preferably, the stent (32) coated with a film is convenient to take out. Preferably, the film may be various flexible materials that meet the human biocompatibility, such as silica gel, fluoroplastic, PE and TPU, with a thickness of 0.01-0.2 mm.

The stent (32) is woven from a single wire. The number of heads (104) of the stent (32) may be preferably 10-30, more preferably 15-25, and most preferably 18-24.

The stent (32) is provided with a developing ring (106) that may be clearly located under the X-ray. The developing ring (106) may be a heavy metal material that is harmless to the human body, such as platinum, gold and tantalum.

In the descriptions of this description, the descriptions with reference to the terms "one embodiment", "some embodiments", "example", "specific example", or "some examples" and the like mean that specific features, structures, materials or characteristics described in conjunction with the embodiments or examples are included in at least one embodiment or example of the present invention. In this description, the schematic representation of the above terms does not necessarily refer to the same embodiment or example. Moreover, the specific features, structures, materials or characteristics described herein may be combined in any suitable modes in any one or more embodiments or examples.

Although the embodiments of the present invention have been shown and described in the foregoing, it can be understood that the foregoing embodiments are illustrative and not construed as limiting the present invention. Those of ordinary skill in the art can make changes, modifications, substitutions and transformations to the foregoing embodiments within the scope of the present invention and without departing from the principle and concept of the present invention. Any modifications, equivalent substitutions, improvements and the like that are made without departing from the principle of the present invention shall all fall within the protection scope of the present invention.

## Claims

1. An antibacterial digestive tract catheter for a gastric diverter, comprising a membrane tube (31), wherein one end of the membrane tube (31) is connected to a stent (32); the membrane tube (31) has an outer diameter of 10-35 mm, and a wall thickness of 0.001-0.3 mm; the membrane tube (31) is fixedly connected to the stent (32) by means of suturing, hot pressing, ultrasonic welding or laser welding; **characterized in that** the membrane tube (31) is made of an antibacterial material; and wherein the antibacterial material is one or more of nano silver ions, silver particles, nano silver, nano copper ions, copper particles, vanillin, an ethyl vanillin compound or chitosan.

2. The antibacterial digestive tract catheter according to claim 1, wherein the membrane tube (31) contains a polymer material, the antibacterial material and a developing material; and wherein the membrane tube (31) is made by uniformly mixing the raw materials and then subjecting to blow molding, extrusion, or mold forming to form a thin film, a tubular film or a thin-wall tube.

3. The antibacterial digestive tract catheter according to claim 1, wherein the membrane tube (31) is a thin film, a tubular film or a thin-wall tube made of the polymer material with the outer surface brushed with the antibacterial material.

4. The antibacterial digestive tract catheter according to claim 1, wherein the polymer material is any one or several of polyethylene, fluorine polymer, polyurethane and silica gel.

5. The antibacterial digestive tract catheter according to claim 1, wherein the developing material is one or more of barium sulfate, bismuth carbonate and a tungsten compound.

6. The antibacterial digestive tract catheter according to claim 1, wherein the stent (32) is a mesh tube woven from a weaving wire (103), and the weaving wire is a biocompatible elastic wire, which may be a metal wire, a polymer material wire or a degradable material wire.

7. The antibacterial digestive tract catheter according to claim 5, wherein the stent (32) includes an upper stent section (101) and a lower stent section (102), and the outer diameter of the upper stent section (101) is 5-15 mm greater than the lower stent section (102).

8. The antibacterial digestive tract catheter according to claim 1, wherein the stent (32) is provided with a developing ring (106) and a withdrawal wire (105).

9. A gastric diverter comprising a shell, a release body, a pushing component and the antibacterial digestive tract catheter according to any one of claims 1 to 8, wherein the shell (1) is tubular; one end of the shell (1) has a first opening (11), and the other end of the shell (1) has a second opening (13); a to-be-released folded catheter is arranged in the shell (1); the release body (2) is arranged at the first opening (11) and connected to one end of the membrane tube (31), and is made of a material that is digested and absorbed or dissolved by a human intestinal tract; the pushing component includes an inner tube (41), a middle tube (42) and an outer tube (43) which are arranged in a sleeved manner in sequence and is able to move relative to each other; one portion of the inner tube (41) is located in the shell (1) and connected to the release body (2) one end of the middle tube (42) extends into the shell (1) through the second opening (13), and is fixedly provided with a stopper piston (12) which is located in the shell (1) and configured for abutting against the membrane tube (31); the outer tube (43) is located outside the shell (1), with one end fixedly connected to the second opening (13); the inner tube (41) moves axially toward an operator to disengage the release body (2) from the shell (1); the inner tube (41) and the middle tube (42) drive the membrane tube (31) to be separated from the shell and spread and then released at designated positions of the human intestinal tract.

## Patentansprüche

1. Antibakterieller Verdauungstraktkatheter für einen Magendivertor, umfassend einen Membranschlauch (31), wobei ein Ende des Membranschlauchs (31) mit einem Stent (32) verbunden ist; der Membranschlauch (31) einen Außendurchmesser von 10-35 mm und eine Wandstärke von 0,001-0. 3 mm aufweist; der Membranschlauch (31) fest mit dem Stent (32) mittels Nähen, Heißpressen, Ultraschallschweißen oder Laserschweißen verbunden ist; **dadurch gekennzeichnet, dass** der Membranschlauch (31) aus einem antibakteriellen Material hergestellt ist; und wobei das antibakterielle Material eines oder mehrere von Nano-Silberionen, Silberpartikeln, Nano-Silber, Nano-Kupferionen, Kupferpartikeln, Vanillin, einer Ethylvanillinverbindung oder Chitosan ist.

2. Antibakterieller Verdauungstraktkatheter nach Anspruch 1, wobei die Membranschlauch (31) ein Polymermaterial, das antibakterielles Material und ein Entwicklungsmaterial enthält; und wobei der Membranschlauch (31) durch gleichmäßiges Mischen der Rohmaterialien und anschließendes Blasformen, Extrudieren oder Formen hergestellt wird, um einen dünnen Film, einen schlauchförmigen Film oder einen dünnwandigen Schlauch zu bilden.

3. Antibakterieller Verdauungstraktkatheter nach Anspruch 1, wobei der Membranschlauch (31) ein dünner Film, ein röhrenförmiger Film oder ein dünnwandiger Schlauch aus dem Polymermaterial ist, dessen äußere Oberfläche mit dem antibakteriellem Material bestrichen ist.

4. Antibakterieller Verdauungstraktkatheter nach Anspruch 1, wobei das Polymermaterial eines oder mehrere von Polyethylen, Fluorpolymer, Polyurethan und Kieselgel ist.

5. Antibakterieller Verdauungstraktkatheter nach Anspruch 1, wobei das Entwicklungsmaterial eines oder mehrere von Bariumsulfat, Bismutcarbonat und einer Wolframverbindung ist.

6. Antibakterieller Verdauungstraktkatheter nach Anspruch 1, wobei der Stent (32) ein Netzschlauch ist, der aus einem Webdraht (103) gewebt ist, und der Webdraht ein biokompatibler elastischer Draht ist, der ein Metalldraht, ein Draht aus Polymermaterial oder ein Draht aus abbaubarem Material sein kann.

7. Antibakterieller Verdauungstraktkatheter nach Anspruch 5, wobei der Stent (32) einen oberen Stentabschnitt (101) und einen unteren Stentabschnitt (102) aufweist und der Außendurchmesser des oberen Stentabschnitts (101) 5 -15 mm größer ist als der untere Stentabschnitt (102).

8. Antibakterieller Verdauungstraktkatheter nach Anspruch 1, wobei der Stent (32) mit einem Entwicklungsring (106) und einem Rückzugsdraht (105) versehen ist.

9. Magendivertor, umfassend eine Schale, einen Auslösekörper, ein Schiebeteil und den antibakteriellen Verdauungstraktkatheter nach einem der Ansprüche 1 bis 8, wobei die Schale (1) rohrförmig ist; ein Ende der Schale (1) eine erste Öffnung (11) und das andere Ende der Schale (1) eine zweite Öffnung (13) aufweist; ein freizusetzender gefalteter Katheter in der Schale (1) angeordnet ist; der Auslösekörper (2) an der ersten Öffnung (11) angeordnet und mit einem Ende des Membranschlauchs (31) verbunden ist und aus einem Material besteht, das von einem menschlichen Darmtrakt verdaut und absorbiert oder aufgelöst wird; das Schiebeteil ein Innenrohr (41), ein Mittelrohr (42) und ein äußeres Rohr (43) umfasst, die in einer geschlitzten Weise hintereinander angeordnet sind und sich relativ zueinander bewegen können; ein Teil des Innenrohrs (41) in der Schale (1) angeordnet und mit dem Auslösekörper (2) verbunden ist, ein Ende des Mittelrohrs (42) durch die zweite Öffnung (13) in die Schale (1) ragt und fest mit einem in der Schale (1) angeordneten und zur Anlage an das Membranschlauch (31) ausgebildeten Anschlagkolben (12) versehen ist; das äußere Rohr (43) sich außerhalb der Schale (1) befindet, wobei ein Ende fest mit der zweiten Öffnung (13) verbunden ist; das Innenrohr (41) sich axial in Richtung eines Bedieners bewegt, um den Auslösekörper (2) von der Schale (1) zu lösen; das Innenrohr (41) und das Mittelrohr (42) das Membranrohr (31) antreiben, um von der Schale getrennt und gespreizt und dann an bestimmten Stellen des menschlichen Darmtrakts freigegeben zu werden.

## Revendications

1. Cathéter antibactérien de tube digestif pour une dérivation gastrique, comprenant un tube à membrane (31), dans lequel une extrémité du tube à membrane (31) est reliée à un stent (32) ; le tube à membrane (31) a un diamètre externe de 10 à 35 mm, et une épaisseur de paroi de 0,001 à 0,3 mm ; le tube à membrane (31) est relié fixement au stent (32) au moyen d'une suturation, d'un pressage à chaud, d'un soudage par ultrasons ou d'un soudage au laser ; **caractérisé en ce que** le tube à membrane (31) est constitué d'un matériau antibactérien ; et dans lequel le matériau antibactérien est un ou plusieurs parmi des nano-ions argent, des particules d'argent, du nano-argent, des nano-ions cuivre, des particules de cuivre, de la vanilline, un composé d'éthyl-vanilline ou du chitosane.

2. Cathéter antibactérien de tube digestif selon la revendication 1, dans lequel le tube à membrane (31) contient un matériau polymère, le matériau antibactérien et un matériau de développement ; et dans lequel le tube à membrane (31) est fabriqué en mélangeant uniformément les matières premières puis en les soumettant à un moulage par soufflage, une extrusion, ou un formage en moule pour former un film mince, un film tubulaire ou un tube à paroi mince.

3. Cathéter antibactérien de tube digestif selon la revendication 1, dans lequel le tube à membrane (31) est un film mince, un film tubulaire ou un tube à paroi mince constitué du matériau polymère avec la surface externe brossée avec le matériau antibactérien.

4. Cathéter antibactérien de tube digestif selon la revendication 1, dans lequel le matériau polymère est l'un quelconque ou plusieurs quelconques parmi polyéthylène, polymère de fluor, polyuréthane et gel de silice.

5. Cathéter antibactérien de tube digestif selon la revendication 1, dans lequel le matériau de développement est un ou plusieurs parmi du sulfate de baryum, du carbonate de bismuth et un composé de tungstène.

6. Cathéter antibactérien de tube digestif selon la revendication 1, dans lequel le stent (32) est un tube à maille tissé à partir d'un fil de tissage (103), et le fil de tissage est un fil élastique biocompatible, qui peut être un fil de métal, un fil de matériau polymère ou un fil de matériau dégradable.

7. Cathéter antibactérien de tube digestif selon la revendication 5, dans lequel le stent (32) comporte une section supérieure de stent (101) et une section inférieure de stent (102), et le diamètre externe de la section supérieure de stent (101) est 5 à 15 mm plus grand que la section inférieure de stent (102).

8. Cathéter antibactérien de tube digestif selon la revendication 1, dans lequel le stent (32) est pourvu d'un anneau de développement (106) et d'un fil de retrait (105).

9. Dérivation gastrique comprenant une enveloppe, un corps de libération, un composant de poussée et le cathéter antibactérien de tube digestif selon l'une quelconque des revendications 1 à 8, dans lequel l'enveloppe (1) est tubulaire ; une extrémité de l'enveloppe (1) a une première ouverture (11), et l'autre extrémité de l'enveloppe (1) a une deuxième ouverture (13) ; un cathéter plié à libérer est agencé dans l'enveloppe (1) ; le corps de libération (2) est agencé au niveau de la première ouverture (11) et relié à une extrémité du tube à membrane (31), et est fabriqué dans un matériau qui est digéré et absorbé ou dissous par un tube digestif humain ; le composant de poussée comporte un tube interne (41), un tube médian (42) et un tube externe (43) qui sont agencés d'une manière emmanchée en succession et aptes à se déplacer les uns par rapport aux autres ; une partie du tube interne (41) est localisée dans l'enveloppe (1) et reliée au corps de libération (2) une extrémité du tube médian (42) s'étend dans l'enveloppe (1) à travers la deuxième ouverture (13), et est pourvu fixement d'un piston d'arrêt (12) qui est localisé dans l'enveloppe (1) et conçu pour venir en butée contre le tube à membrane (31) ; le tube externe (43) est localisé à l'extérieur de l'enveloppe (1), avec une extrémité reliée fixement à la deuxième ouverture (13) ; le tube interne (41) se déplace axialement en direction d'un opérateur pour désaccoupler le corps de libération (2) par rapport à l'enveloppe (1) ; le tube interne (41) et le tube médian (42) entraînent le tube à membrane (31) pour qu'il se sépare de l'enveloppe et s'étale puis se libère au niveau de positions désignées du tube digestif humain.
